# EUROPEAN PATENT SPECIFICATION

(11) **EP 0 401 362 B1**
(45) Date of publication and mention of the grant of the patent: **29.05.1996**
(21) Application number: 90901476.3
(22) Date of filing: 22.12.1989
(51) Int. Cl.: C12N 9/24, C12N 15/52, C12N 15/56, C12N 15/63, C12N 15/79, C12N 15/81, C12N 15/85

(54) **CHO CELLS PRODUCING ENZYMATICALLY ACTIVE RECOMBINANT GLUCOCEREBROSIDASE**
CHO-ZELLEN BEFÄHIGT ENZYMATISCH AKTIVE REKOMBINANTE-GLUKOCEREBROSIDASE ZU PRODUZIEREN
CELLULES CHO CAPABLE D'EXPRIMER GLUCOSYL-CERAMIDASE DE RECOMBINAISON A ACTION ENZYMATIQUE

(30) Priority: 23.12.1988 US 289589
(43) Date of publication of application: 12.12.1990
(73) Proprietor: GENZYME CORPORATION, Boston Massachusetts 02111 (US)
(72) Inventor: RASMUSSEN, James,C/O Genzyne Corporation, Cambridge, MA 02139 (US); BARSOMIAN, Gary, Georgetown, MA 01830 (US); BERGH, Michel, Belmont, MA 02178 (US)
(74) Representative: Deans, Michael John Percy
(86) International application number: US8905801
(87) International publication number: WO9007573

(56) References cited:
- WO-A-89/05850
- US-A- 4 745 051
- DNA, vol. 7, no. 2, 1988; B.M. MARTIN et al., pp. 99-106
- MOLECULAR & BIOLOGICAL MEDICINE, vol. 3, no. 3, 1986; P.V. CHOUDARY et al., pp. 293-299
- ENZYME, vol. 41, no. 3, 1989; G.A. GRABOWSKI et al., pp. 131-142
- PROCEEDINGS OF THE NATL. ACADEMY OF SCIENCE USA, vol. 84, February 1987, Washington, DC (US); SORGE et al., pp. 906-909
- JOURNAL OF BIOLOGICAL CHEMISTRY, vol. 259, February 1984; HSIEH et al., pp. 2375-2382
- DNA, "Cloning-A Practical Approach", vol. 11, July 1985, IRL Press, Washington, DC (US); C. GORMAN, pp. 143-165
- COLD SPRING HARBOR SYMPOSIA ON QUANTITATIVE BIOLOGY, vol. L1, 1986, Cold Spring Harbor, NY (US); CHAUDARY et al., pp. 1047-1052
- MOLECULAR & CELLULAR BIOLOGY, vol. 1, September 1981; SUBRAMANI et al., pp. 854-864
- METHODS IN ENZYMOLOGY, vol. LVIII, March 1979, Academic Press, New York, NY (US); HAM et al., pp. 72-74

## Description

This invention relates to expression of enzymatically active recombinant glucocerebrosidase.

Gaucher's disease is an autosomal recessive lysosomal storage disorder characterized by a deficiency in a lysosomal enzyme, glucocerebrosidase ("GCR"), which hydrolyzes the glycolipid glucocerebroside. In Gaucher's patients, deficiency in this enzyme causes the glycolipid glucocerebroside, which arises primarily from degradation of glucosphingolipids from membranes of white blood cells and senescent red blood cells, to accumulate in large quantities in lysosomes of phagocytic cells, mainly in the liver, spleen and bone marrow. Clinical manifestations of the disease include splenomegaly, hepatomegaly, skeletal disorders, thrombocytopenia and anemia.

Current treatments for patients suffering from this disease include administration of analgesics for relief of bone pain, blood and platelet transfusions, and in severe cases, splenectomy. Joint replacements may be necessary for patients who experience bone erosion. Brady, 1966, 275 New England Journal of Medicine 312, proposed enzyme replacement therapy with GCR as a treatment for Gaucher's disease. However, Furbish et al., 1978, 81 Biochem. Biophys. Research Communications 1047, observed that infused human placental GCR does not reach the site at which it is active, namely lysosomes of cells of the reticuloendothelial system, but rather is taken up by hepatocytes. Furbish et al., 1981, 673 Biochem. Biophys. Acta 425, improved delivery of human placental GCR to phagocytic cells by treating the GCR sequentially with neuraminidase, β-galactosidase and β-N-acetylhexosaminidase, and demonstrated that the treated GCR was taken up more efficiently by rat Kupffer cells than untreated protein.

Sorge et al., 1985, 82 Proc. Nat'l. Acad. Sci, USA 7289, and Tsuji et al., 1986, 261 J. Biol. Chem. 50 describe cloning and sequencing of a gene encoding human GCR.

In general, we describe recombinant enzymatically active GCR produced by a Chinese hamster ovary (CHO) cell. The term "recombinant GCR" ("rGCR") is used herein to mean any GCR produced from genetically manipulated GCR-encoding nucleic acid inserted into a cell. The nucleic acid is generally placed within a vector, such as a plasmid or virus, as appropriate for the host cell. The term "enzymatically active" is used herein with respect to recombinant GCR to mean that the rGCR is able to hydrolyze a glucocerebroside, and can cleave the low molecular substrate 4-methyl-umbelliferyl-β-D-glucoside with an activity of at least 10⁶ units per milligram of rGCR.

We describe CHO cells producing recombinant enzymatically active GCR having at least one exposed mannose residue, wherein the GCR is capable of specifically binding with a human mannose receptor protein.

The term "GCR having at least one exposed mannose residue" means that the GCR is glycosylated and at least one of the carbohydrate groups attached to the GCR has a carbohydrate chain terminating with a mannose residue. Preferably, the exposed mannose residue is readily available to bind with a mannose receptor protein, the exposed mannose receptor being positioned external to the GCR in its three dimensional configuration. A GCR that is "capable of specifically binding with a human mannose receptor protein", as that term is used herein is a GCR that is specifically recognized by the receptor protein to an exposed mannose residue.

The rGCR preferably has an amino acid sequence with at least 95% homology to an amino acid sequence of a human GCR; the rGCR has at least two exposed mannose residues; the rGCR includes a carbohydrate moiety having between 3 and 9 mannose residues; preferably the mannose residues are arranged in a Man₃ to Man₉ structure (referred to as Man₃GlcNAc₂, etc.; the receptor protein naturally occurs in a phagocytic cell.

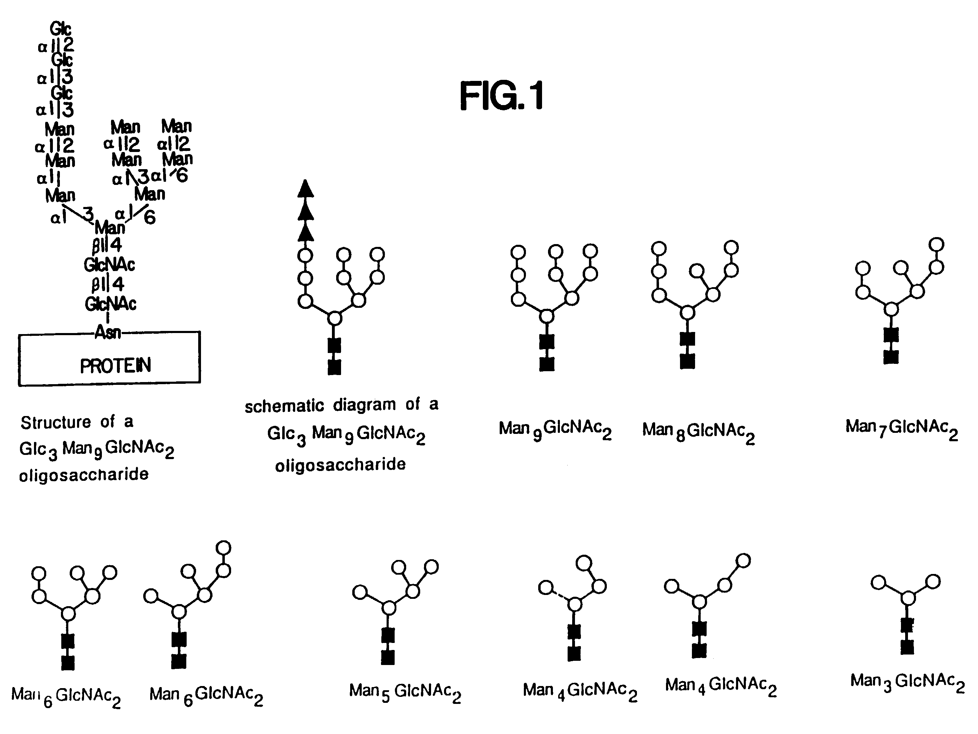

Fig. 1 shows as examples a variety of carbohydrate moieties having between 3 and 9 mannose residues arranged in a Man₃ to Man₉ structure. The term "Man₃ to Man₉ structure", as used herein refers to arrangements of mannose residues such as are shown in Fig. 1. and their structural isomers.

In a first aspect of the invention, there is provided a Chinese hamster ovary (CHO) cell comprising nucleic acid encoding enzymatically active glucocerebrosidase, wherein said glucocerebrosidase is capable of specifically binding with a human mannose receptor protein.

In preferred embodiments, the nucleic acid is a vector including DNA encoding an amino acid sequence of a naturally occurring human GCR.

In other preferred embodiments, the nucleic acid is DNA lacking at least 50% of the region that is present in a naturally occurring GCR gene between the promoter of the GCR coding sequence and the ATG start site of the gene.

In other preferred embodiments, the nucleic acid is DNA present in the plasmid pGB20, or in the plasmid pGB37, or in the plasmid pGB42; and the cell is a Chinese hamster ovary cell, transformed with any of such plasmids or cotransformed with plasmid pGB34 and any of such plasmids.

In another aspect, the invention provides a method for producing enzymatically active glucocerebrosidase comprising the steps of: introducing nucleic acid encoding human glucocerebrosidase into a CHO cell; causing said cell to express and secrete said glucocerebrosidase into a culture medium; and purifying said glucocerebrosidase from said culture medium. Expressed rGCR that is secreted by the cell into the surrounding medium can be purified directly from the medium.

The invention provides CHO cells producing enzymatically active rGCR in a form that is specifically recognised by human mannose receptor proteins. The rGCR thus obtained is suitable for administration to a human suffering from Gaucher's disease using a standard enzyme replacement protocol, because the enzyme is free from viral or bacterial agents commonly found in human tissues such as, for example, human placenta, from which GCR is conventionally derived. In addition, the rGCR produced in CHO cells is secreted in large amounts from said cells into the surrounding medium, from which it is readily purified.

Other features and advantages of the invention will be apparent from the following description of the preferred embodiments thereof, and from the claims.

### Description of the Preferred Embodiments

The drawings will first briefly be described.

### Drawings

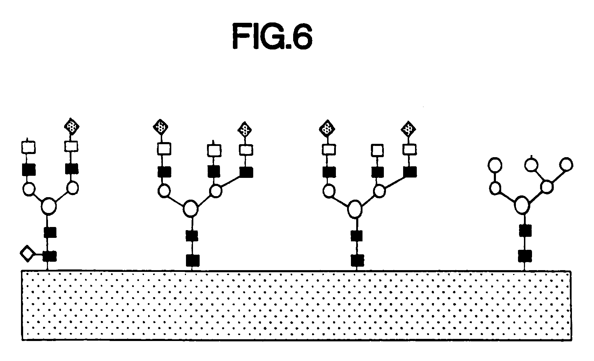

Fig. 1 and Fig. 6 are diagrammatic representations of various forms of mannose-terminating carbohydrate moiety of rGCR and of a typical complex-type carbohydrate moiety; circles represent mannose residues, black triangles represent glucose residues, black squares represent N-acetylglucosamine residues, open squares represent galactose residues, diamonds represent N-acetyl neuraminic acid residues, shaded triangles represent fucose;
Fig. 2 is a schematic representation of the spatial relationships in various clones of the GCR gene among the GCR encoding regions, the 5' and the 3' noncoding regions and the polyhedrin regulatory sequences;
Fig. 3 is a schematic representation of construction of a deletion version of GCR (with a 5' noncoding region removed and a portion of the 3' noncoding region removed) inserted within a plasmid, namely plasmid pGB9.D21;
Fig. 4 is a schematic representation of construction of plasmid pVL941.GCRD21;

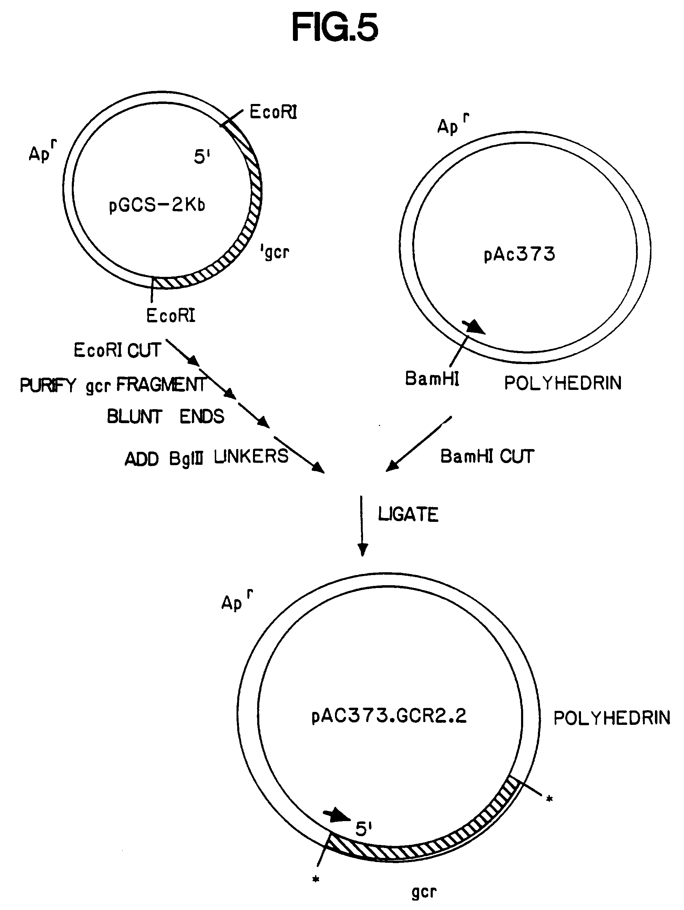

Fig. 5 is a schematic representation of construction of plasmid pAc373.GCR2.2;
Fig. 7 is a schematic representation of construction of plasmids pGB9.D21C and pGB9.D21C1;
Fig. 8 is a schematic representation of construction of plasmid pGB20.
Fig. 9 is a schematic representation of construction of plasmid pGB34.
Fig. 10 is a schematic representation of construction of plasmids pGB37 and pGD42.

### Glucocerebrosidase (GCR)

As defined above, GCR has an enzyme activity which causes hydrolysis of a glucocerebroside.

A gene encoding this enzyme activity has been cloned as described above, and its DNA sequence is known. In the present application, applicants provide examples of use of this cloned DNA to cause production of rGCR in CHO cells having a structure suitable for therapeutic use in humans.

The invention is hereinafter described by way of example only.

There follows a description of examples of insertion of human GCR-encoding DNA into mammalian vectors and expression of the DNA by mammalian cells.

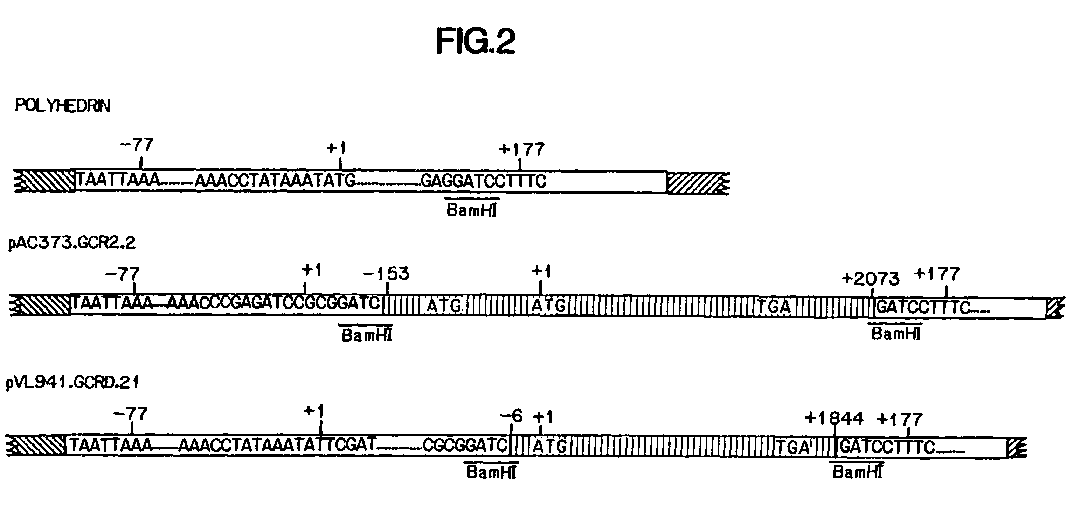

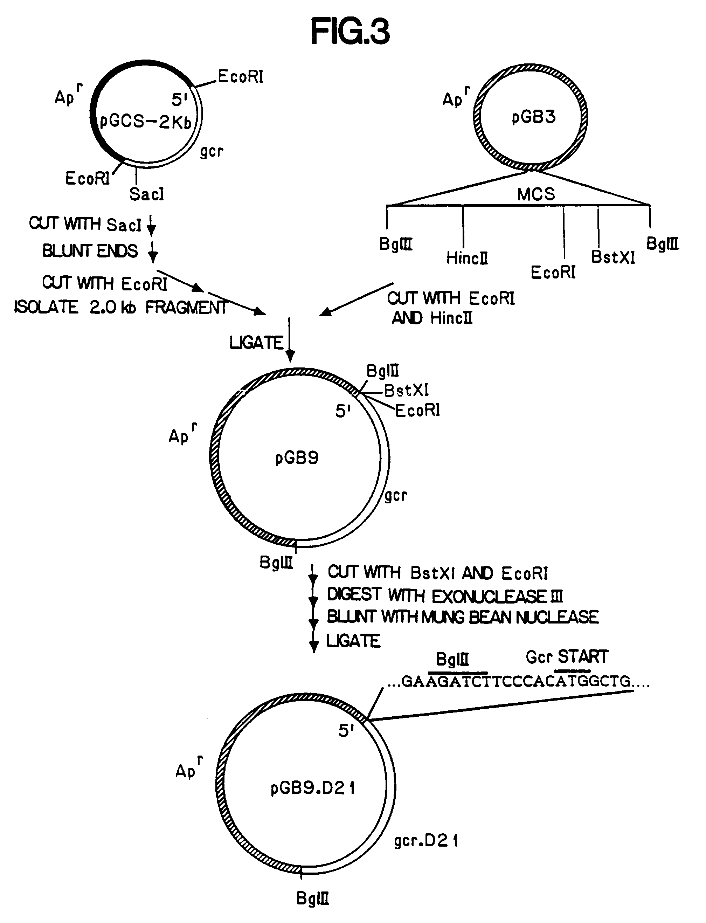

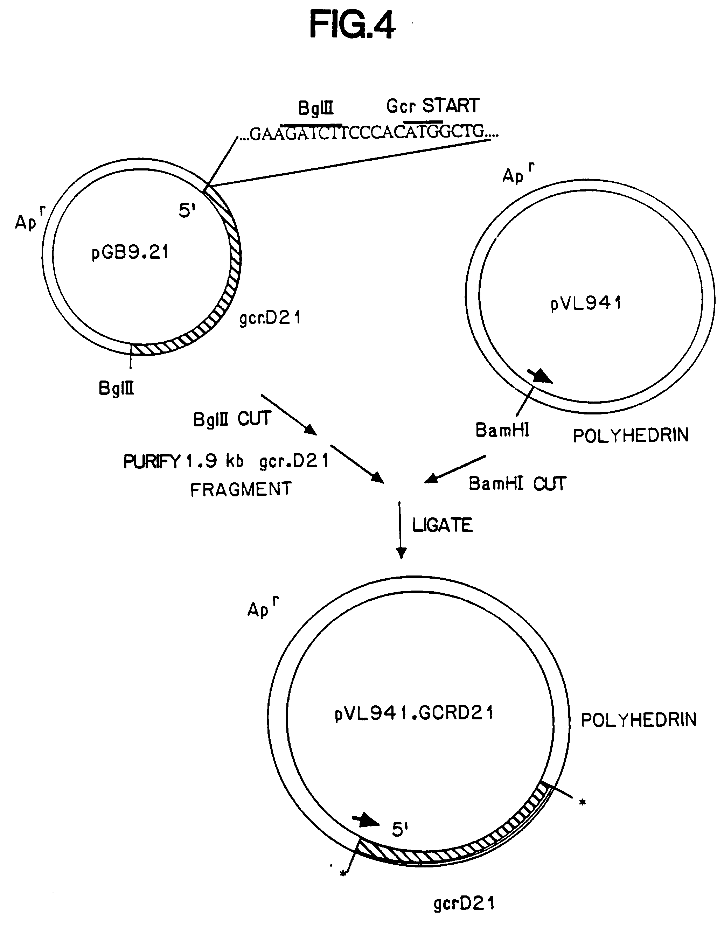

Referring to Figs. 2, 3 and 4, we constructed the plasmid pGB9.D21 (Fig. 3), which carries a deletion derivative, GCR.D21, bounded by BglII sites. This BglII cassette carrying the GCR gene has 5 bases at the 5' end and 290 bases at the 3' end between the cloning (BglII) ends and the GCR coding sequences, compared to 160 and 500 bases, respectively, in the original cDNA pGCS-2kb with EcoRI cloning ends. The stepwise construction of pGB9.D21 is summarized in Fig. 3. Briefly, the GCR cDNA clone, pGCS-2kb, was cleaved approximately 290 base pairs from the 3' end of the GCR coding sequence with SacI and the ends blunt-ended with T4-DNA polymerase. A second cut, approximately 160 bp from the 5' end of GCR, with EcoRI created a 2.0 kb fragment containing the GCR coding sequence. This fragment was purified and ligated to HincII and EcoRI cut pGB3 to form pGB9. E. coli plasmid pGB3 was constructed from pBluescript SK+ (Stratagene, La Jolla, CA) by independently blunting the SacI and ApaI sites with T4 polymerase and ligating in BglII linkers. The resulting plasmid, pGB3, contains BglII linkers at either end of its multiple cloning site and retains lacZ activity in the host XL-1 Blue. pGB9 was digested with BstXI, EcoRI, and then with exonuclease III and mung bean nuclease to remove excess 5' non-coding sequence between the BglII site and the 5' end of the GCR coding sequence. After this treatment the ends were religated, and the plasmids were used to transform E. coli XL-1 blue. Transformants were screened using restriction analysis, and then DNA sequencing was used to determine the lengths of the deletions. Using this approach we identified pGB9.D21, which has only 5 bp between the BglII site and the start codon of GCR.

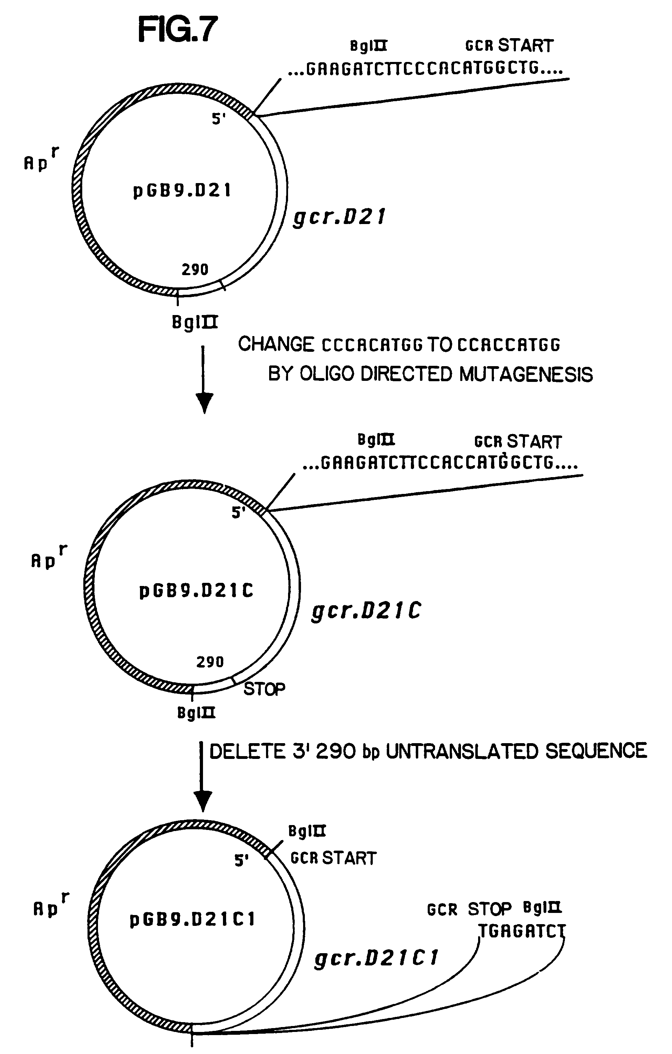

To optimize expression of GCR in mammalian cells, we further modified the shortened version of the GCR-encoding fragment GCR.D21 BglII cassette containing the gcr gene. In general, with reference to Fig. 7, the modifications were made using oligonucleotide directed mutagenesis (described generally in Kunkel, 1986, 82 Proc. Natl. Acad. Sci. U.S.A. 488-492; Wang et al., 1989, 7 Biotechniques, 1000-1010) to alter the nucleotide sequence near the GCR translation start to match the consensus sequence (CCACCATGG) for optimal translation in mammalian cells (as described in Kozak, 1986, 44 Cell 283-292); and to delete the excess sequence 3' of the gcr.D21C stop codon. The excess 3' sequence deletion removes potential message destabilizing sequences, and permits modulation of DHFR expression relative to GCR from bicistronic vectors.

### Vector constructions

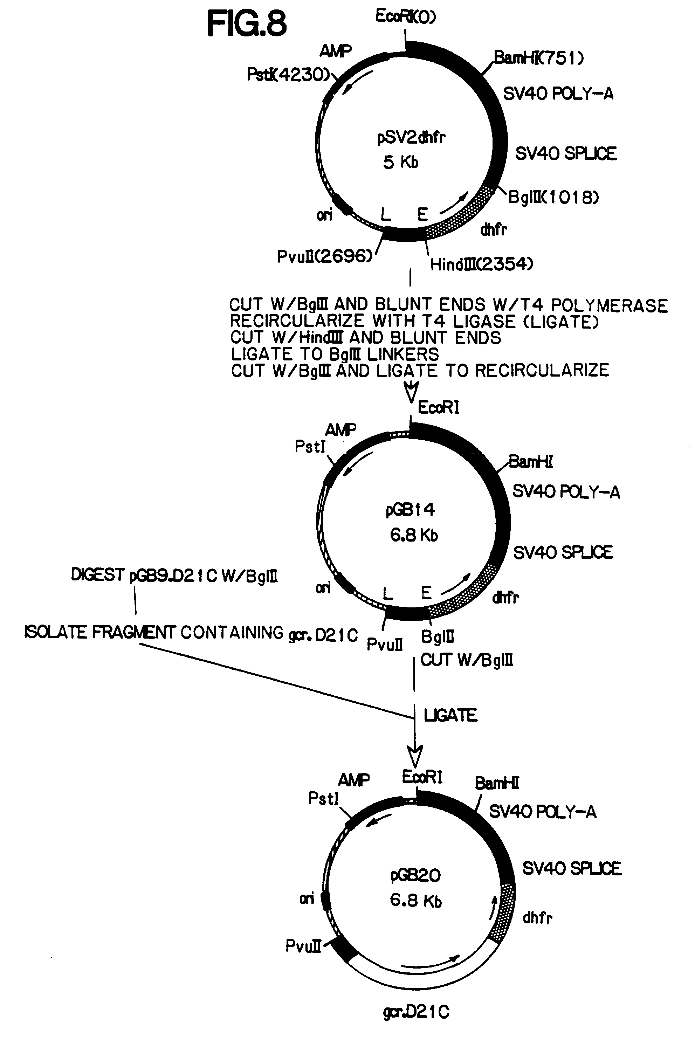

A bicistronic gcr-dhfr expression vector for CHO cells was constructed as shown in Fig. 8 from the vector pSV2-dhfr (described in Subramani et al., 1981, 9 Molecular and Cellular Biology 854-864). This vector, pGB20, contained gcr.D21C followed by dhfr under the control of the SV40 early promoter. DHFR expression depends upon ribosomes translating the dhfr after translating gcr, which is located adjacent at the 5' end of the dhfr message. This bicistronic arrangement reduced levels of expression of DHFR relative to GCR. A resulting gain in GCR expression relative to DHFR can be realized after stable transfectants have been selected using methotrexate.

A second series of bicistronic gcr expression vectors were constructed having the following characteristics: 1) transcription of gcr driven by a SV40 enhancer-Adenovirus major late promoter (Ad MLP) combination; 2) transcription of gcr in a bicistronic arrangement with dhfr; 3) termination and polyadenylation signals from SV40; and 4) a minimal 2 kb segment of DNA containing a prokaryotic origin and ampicillin resistance gene, but lacking the "poison" sequences which can be deleterious in mammalian cells (Lusky and Botchan, 1981, 293 Nature 79-81).

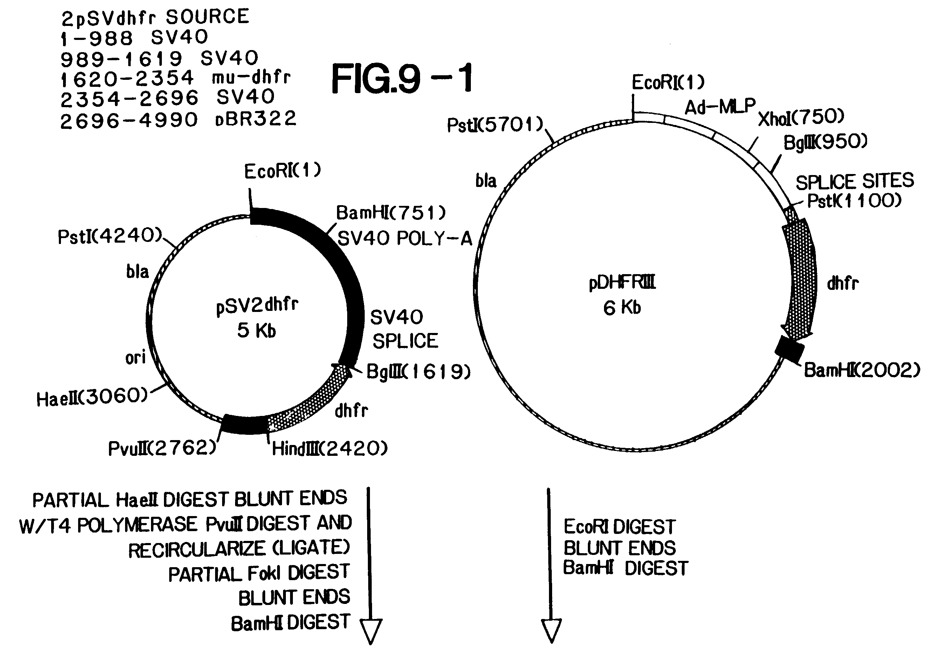

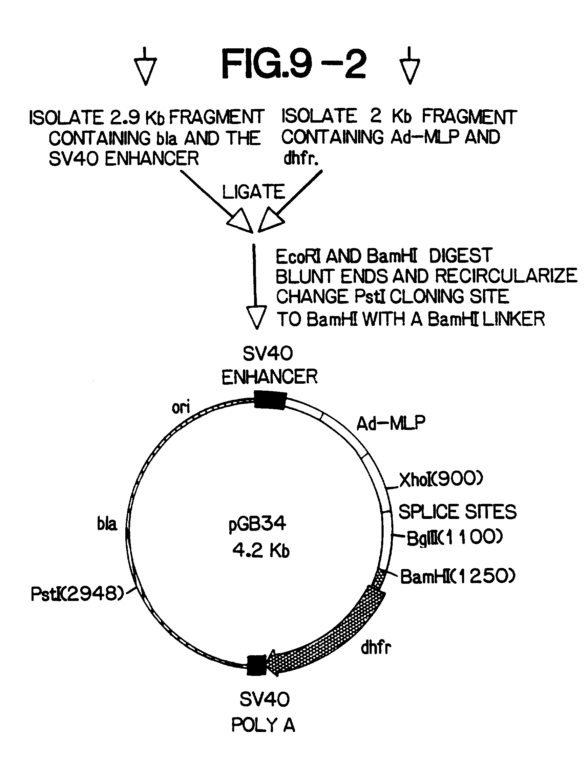

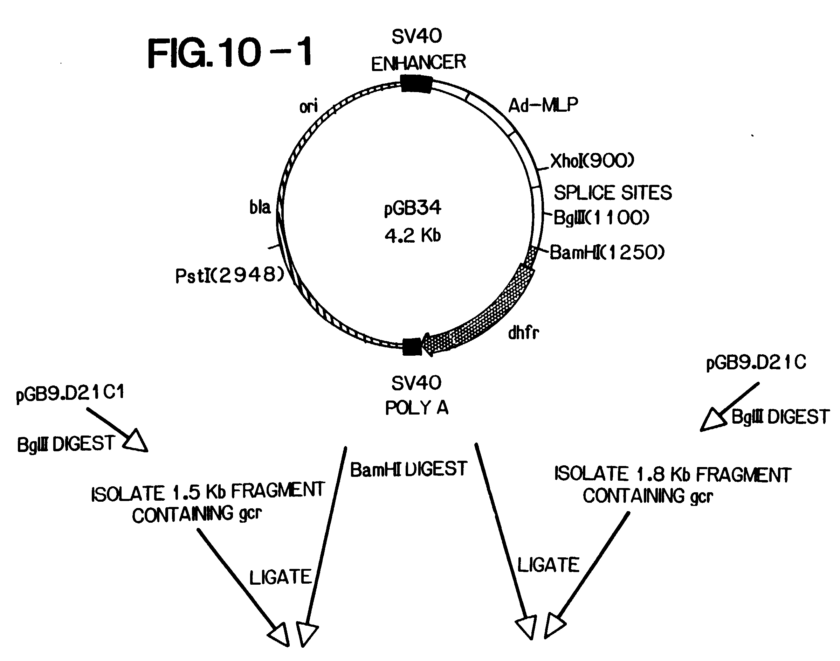

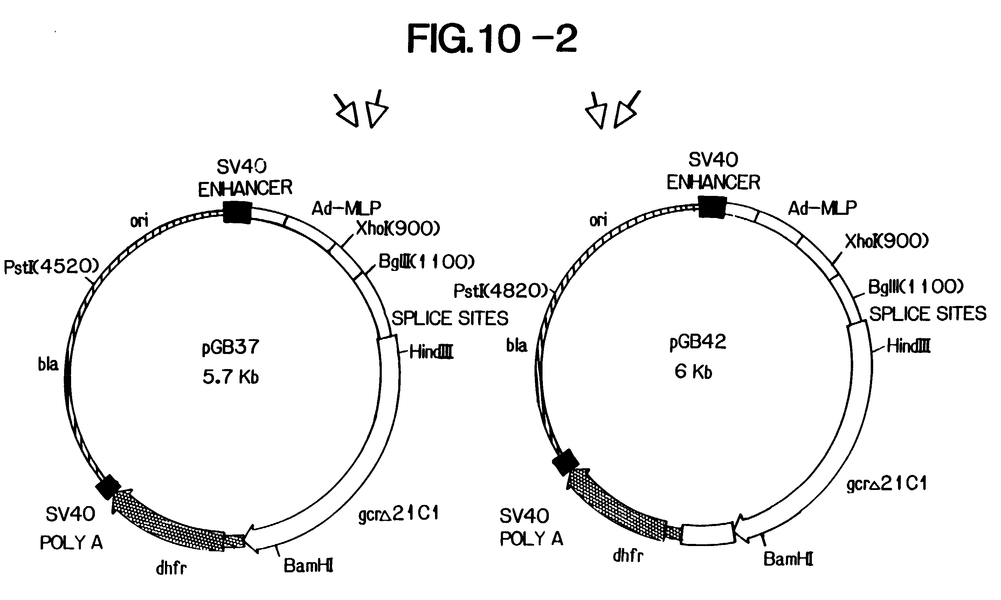

The base vector, pGB34, was constructed from pSV2dhfr as shown in Fig. 9. In a first step "poison" sequences between the PvuII and MaeII sites which might adversely affect expression in the final vector were removed. The resulting plasmid carries the SV40 enhancer (72 bp repeat), the pBR322 replication origin and the ampicillin resistance coding gene (bla) within a minimal fragment (2 kb) between the FokI and EcoRI sites. In a second step the 2 kb FokI-EcoRI fragment combined with a fragment from pDHFRIII (described in Berkner and Sharp, 1985, 13 Nucleic Acids Research 841-857) containing the Ad-MLF and the dhfr gene and the SV40 polyadenylation signal. Excess sequence between the polyadenylation site and the pBR322 sequences were removed and the PstI cloning site was changed to BamHI by standard procedures using BamHI linkers. The resulting construct, pGB34, carries very little sequence in excess of that required for efficient production of recombinant proteins in mammalian cells. Versions of this vector were constructed using either gcr.D21C or gcr.D21C1 (shown in Fig. 7) for expression of GCR in CHO cells, as shown in Fig.10. These constructs express DHFR at different levels, thus modulating DHFR selection and the relative amplification of GCR expression. These constructs can be used by themselves as bicistronic amplifiable gcr expression vectors; alternatively, the more widely used scheme of cotransfection using pGB34 can be used.

### Transfecting Mammalian Cells

Any of several procedures can be used for introducing the vector DNA into mammalian cells, including calcium phosphate transfection and electroporation (described generally in F.M. Ausubel et al*.*, eds., 1989, Current Protocols in Molecular Biology, pages 9.1.1-9.1.4 and 9.3.1-9.3.2, respectively, Wiley & Sons, New York), Lipofectin™ transfection (as described by the manufacturer, Bethesda Research Laboratories, Gaithersburg, MD), protoplast fusion (as described, e.g., in Sandri-Goldin et al., 1981, 1 Mol. Cell. Biol. 743-52, or polybrene transfection. Selection of recombinant colonies is performed by incubating the cells in culture medium devoid of ribonucleosides and deoxyribonucleosides, containing dialyzed fetal calf serum.

### Amplifications

To increase expression of rGCR the cells can be subjected to an amplification procedure similar to the one described in F.M. Ausubel et al., eds., 1989, Current Protocols in Molecular Biology, pages 9.9.1-9.9.6, Wiley & Sons, New York), which is similar to the procedure described in R.J. Kaufman et al., 1982, 159 J. Cell. Mol. Biol. 601-21. In general, higher levels of expression result from higher levels of methotrexate resistance. Other amplification procedures can be utilized as well, by using other amplifiable genes instead of the dhfr gene. Examples are the ornithine decarboxylase gene, the adenosine deaminase gene, and others, as well as combinations thereof, known to persons of ordinary skill. Examples have been reviewed by R.J. Kaufman in J. Setlow, Ed., 1989, 9 Genetic Engineering 155-198, Plenum Press, New York. In order to get amplification using other genes, the dhfr gene in the vectors described above is replaced by the gene of choice, e.g., the ornithine decarboxylase gene or the adenosine deaminase gene; after transfection with such a vector, amplification can be obtained using the appropriate selective medium. Assays

Enzymatic activity of recombinant GCR expressed in chinese hamster ovary cells was measured using 4-methyl-umbelliferyl-B-D-glucoside as a substrate. Enzymatic hydrolysis of this substrate generates a fluorescent product, which is quantitated using a spectrofluorometer. Details of this procedure are described in Methods of Enzymology, Vol. L, pp. 478-79, 1978. The assay is caried out under conditions in which other, non-GCR glucosidase activities are partially inhibited, i.e., by using a phosphate buffer, pH 5.9, 0.125 % taurocholate, 0.15 % Triton X-100.

Another way to establish the presence of recombinant GCR is by using a polyclonal antibody raised in rabbits against a preparation of glucocerebrosidase purified from human placenta. The medium or the cell lysate from GCR producing cells can be subjected to SDS-polyacrylamide gel electrophoresis, after which proteins are transferred from the gel to nitrocellulose. The presence of rGCR on nitrocellulose is established by probing with the antibody, which is detected by standard techniques using the biotinylated protein A-alkaline phosphatase-conjugated streptavidin technique, or biotinylated goat-anti-rabbit IgG antibody-alkaline phosphatase-conjugated streptavidin, or any other standard method for detection of antibody on nitrocellulose. Details of such procedures have been described in, for example, E. Harlow and D. Lane, Eds., Antibodies, a Laboratory Manual, Cold Spring Harbor Laboratory, NY, 1988.

Another way of determining production of rGCR by CHO cells is by labeling the growing cells in vivo with [35S]methionine, and then harvesting the medium and lysing the cells, e.g., in a buffer containing 50 mM citrate pH 6.5, 1% sodium cholate, at intervals after labeling. The lysate and medium are then immunoprecipitated using the polyclonal antibody according to standard procedures. The polyclonal antibody can be used in various forms, such as, e.g., in the form of antiserum, or purified on protein A-agarose or protein G-agarose, or affinity-purified over a matrix onto which glucocerebrosidase has been immobilized. A higher purity of the antibody generally results in a lower background signal. Alternatively, a monoclonal antibody against GCR can be used. Examples of these detection procedures have been described in, for example, E. Harlow and D. Lane, Eds., Antibodies, a Laboratory Manual, Cold Spring Harbor Laboratory, NY, 1988.

These detection methods demonstrated expression of rGCR in CHO cells. In a typical run, cells that were transfected with one of the vectors described above, either using the dicistronic approach or the co-transfection procedure, expressed rGCR at levels between 1-10 mg/L, varying with cell density, culture conditions and cell support matrix. The rGCR is found both intracellularly and in the medium. The intracellular rGCR is sensitive to endoglucosaminidase H and to endoglucosaminidase F, indicating that the carbohydrate chains on the protein are most likely of the so-called high-mannose type, and thus the intracellular rGCR is particularly useful for treatment of Gaucher's disease, as described below. The rGCR recovered from the medium is larger in molecular weight, and is resistant to endoglucosaminidase H.

### Carbohydrate Structure of rGCR

To determine whether a rGCR produced as described above has been correctly glycosylated to be useful for treatment of Gaucher's disease the following procedure can be performed to determine the carbohydrate structure, and in particular the mannose configuration within the carbohydrate structure, of the rGCR. Generally, the carbohydrate should contain at least one exposed mannose and preferably has a Man₃-Man₉ structure, or a structure with the same functional features as a carbohydrate having a Man₃-Man₉ structure (e.g., other sugar residues can be substituted for one or more of the sugar residues shown). There are generally four oligosaccharide moieties in human placental GCR. Recombinant GCR having these moieties present in a Man₃-Man₉ structure has greater affinity than unglycosylated GCR for the mannose receptor in humans. The more mannose residues per oligosaccharide moiety, and the more such moieties, the greater this affinity. Recombinant GCR according to this invention has at least one such oligosaccharide with at least one exposed mannose, but preferably has two, three, or four such oligosaccharides each with a Man₃-Man₉ structure.

Analysis of oligosaccharides derived from rGCR generally followed the procedure described by Hirani et al., 162 Anal. Biochem. 485, 1987. Asn-linked oligosaccharides were released from SDS-denatured rGCR (100 ug) by incubation with N-glycanase enzyme (80 units) at 37°C for 18 hours. The completeness of the reaction was judged by SDS-polyacrylamide gel electrophoresis. The liberated oligosaccharides were recovered in the supernatant after precipitating the protein with ethanol (75% v/v) and radiolabeled at the reducing end by treatment with sodium borotritide. Labeled oligosaccharides were analyzed by a combination of high performance liquid chromatography (hplc) and exoglycosidase digestion.

The degree of sialylation was determined by analyzing the tritium-labeled oligosaccharides by hplc on a Micropak AX-10 column pre-equilibrated in 25 mM KH₂PO₄, titrated to pH 4.0 with phosphoric acid. GCR with high sialic acid content is unlikely to useful in this invention, but GCR with a low sialic acid content, i.e., uncharged GCR, is more likely to have exposed mannose moieties. The column was eluted with the same buffer for 15 minutes and then for 30 minutes using a linear gradient of 25 mM KH₂PO₄, pH 4.0, to a final concentration of 500 mM KH₂PO₄,pH 4.0. In this elution protocol, oligosaccharides eluted from the column in characteristic positions depending upon the number of attached sialic acid residues. The Asn-linked oligosaccharides derived from recombinant GCR consisted primarily (95%) of neutral species. Such neutral species are potentially useful in this invention.

The size of each neutral and/or desialylated oligosaccharide was analyzed by hplc using a Micropak AX-5 column. The column was pre-equilibrated with acetonitrile:water (65:35) and elution was performed using a 60 minute gradient in which the water content of the solvent increased at the rate of 0.5%/minute. This procedure fractionates oligosaccharides according to size. The column was calibrated with oligosaccharide standards having the structures shown below. Analysis of the oligosaccharides derived from rGCR obtained from infected SF9 cells showed that they consisted of a single species with a retention time similar to Man₃GlcNAc(FUc)GlcNAc_{0T}.

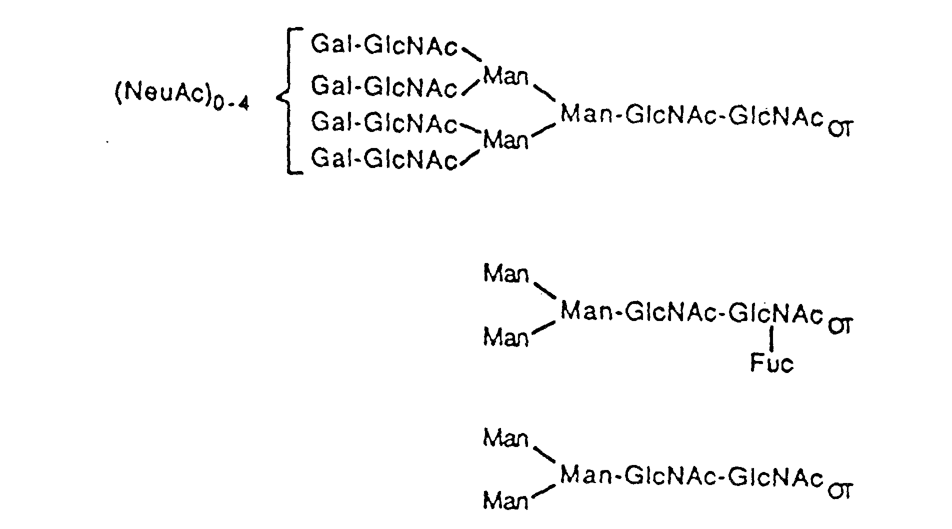

A presence of exposed mannose groups is readily determined by treatment of these oligosaccharides with mannosidases which specifically remove mannose groups. Such treatment, and the analysis of the results, is performed by standard procedures.

In another method for determining the usefulness of an rGCR, the ability of the rGCR to bind to and be taken up by macrophages is measured. This targeting of rGCR to macrophages is mediated by the Man/GlcNAc receptor and can be determined using thioglycollate-elicited peritoneal macrophages obtained from mice, as described by Stahl et al., 93 J. Cell Biol. 49, 1982. Briefly, mice (25-30 g, C57 strain) are injected intraperitoneally with 1-1.5 ml thioglycollate broth (Difco, Detroit, MI). After 3-4 days the mice are sacrificed by suffocation in CO₂ and the peritoneal cavity rinsed with phosphate buffered saline. The cells are pelleted by centrifugation (500 g, 10 min), resuspended in DME (GIBCO, Grand Island, NY) containing 10% fetal calf serum, and plated in 96-well tissue culture plates. After 90 min. non-adherent cells are washed away, and the adherent macrophages are incubated for specified time periods, ranging from 0 to 180 minutes in culture medium containing specified quantities of rGCR, ranging from 0 to 20 ug in 200 ul at a temperature of 37°C, in the absence and presence of yeast mannan (2-10 mg/ml). After incubation, the medium containing excess rGCR is removed, the cells are washed several times and then lysed, and the amount of rGCR taken up by the cells is determined in the cell lysate. The amount taken up in the presence of yeast mannan is non-specific uptake. The difference between the two values is the amount taken up specifically via the Man/GlcNAc receptor. No uptake occurs when the experiment is done at 4°C, but the rGCR binds to the cell surface. In this way, Man/GlcNAc receptor-specific binding of rGCR can be determined.

### Use

The rGCR produced is CHO cells is useful for therapeutic treatment of Gaucher's disease by providing a therapeutic amount of the rGCR. By therapeutic amount is meant an amount of rGCR which will cause significant alleviation of clinical symptoms of Gaucher's disease. Such rGCR must be post-translationally modified, as described above, to provide a carbohydrate structure which will target to human mannose receptors.

Generally, such rGCR has at least two carbohydrate moieties each having a Man₃-Man₉ structure, and such rGCR represents at least 50% of the rGCR provided in the therapeutic composition. For example, provision of between 10 and 500 milligrams per 70 kg patient per month to provide that patient with between 0.25 and 3 grams rGCR over a one year period. The rGCR is provided in the form of a pharmaceutical composition in any standard pharmaceutically acceptable carrier, such as physiological saline, and is administered by any standard procedure, for example by intraveneous injection.

### Deposit

An E. coli strain (DH5α) harboring the plasmid pVL941.GCRD21 was deposited on December 22, 1988 with the American Type Culture Collection and was assigned ATCC accession number 67,866. An E. coli strain [??] harboring the plasmid pGB42 was deposited on December 21, 1989 with the American Type Culture Collection and was assigned ATCC accession number .

Applicants and their assignee, Genzyme Corporation, acknowledge their responsibility to replace these cultures should they die before the end of the term of a patent issued hereon, 5 years after the last request for a culture, or 30 years, whichever is the longer, and their responsibility to notify the depository of the issuance of such a patent, at which time the deposit will be made irrevocably available to the public. Until that time the deposit will be made available to the Commissioner of Patents under the terms of 37 C.F.R. §1-14 and 35 U.S.C. §112.

### Other Embodiments

Other embodiments are within the following claims. For example, rGCR having an appropriate carbohydrate structure can be produced by introducing GCR-encoding DNA into CHO cells and treating that cell during its growth with inhibitors of carbohydrate processing such as deoxy-mannojirimycin, swainsonine, castanospermine, deoxy-nojirimycin, N-methyl-deoxy-nojirimycin, or their equivalent inhibitors. These inhibitors act to inhibit specific steps in the conversion of Glc₃Man₉GlcNAc₂ to smaller species shown in the figure, thus, providing a greater number of exposed mannose residues.

## Claims

1. A Chinese hamster ovary (CHO) cell comprising nucleic acid encoding enzymatically active glucocerebrosidase, wherein said glucocerebrosidase is capable of specifically binding with a human mannose receptor protein.

2. A CHO cell according to Claim 1, wherein said nucleic acid comprises DNA encoding human glucocerebrosidase.

3. A CHO cell according to Claim 1, wherein said DNA lacks at least 50% of a naturally occurring region between the promoter of said glucocerebrosidase-encoding DNA and the ATG start site of said glucocerebrosidase-encoding DNA.

4. A method for producing enzymatically active glucocerebrosidase comprising the steps of: introducing nucleic acid encoding human glucocerebrosidase into a CHO cell; causing said cell to express and secrete said glucocerebrosidase into a culture medium; and purifying said glucocerebrosidase from said culture medium.

5. A CHO cell according to Claim 1 wherein said cell is transformed with any plasmid selected from the group pGB20 having a restriction map as shown in Figure 8, pGB37 having a restriction map as shown in Figure 10, and pGB42 having a restriction map as shown in Figure 10.

6. A CHO cell according to Claim 1 wherein said cell is cotransformed with plasmid pGB34 having a restriction map as shown in Figure 9, and any plasmid selected from the group pGB20 having a restriction map as shown in Figure 8, pGB37 having a restriction map as shown in Figure 10, and pGB42 having a restriction map as shown in Figure 10.

7. The method of Claim 4 wherein the pH of said culture medium is between about pH 6.5 and pH 7.2.

8. The method of Claim 4 wherein pH of said culture medium is between about pH 6.6 and pH 6.8.

9. The method of Claim 4 wherein said culture medium contains 0₂ in an amount below about 50% saturation and sufficient to maintain the cells.

10. The method of Claim 4 wherein said culture medium contains 0₂ in an amount between about 20% saturation and about 30% saturation.

11. A method according to Claim 4 wherein said nucleic acid is a plasmid selected from the group of pGB20 having a restriction map as shown in Figure 8, pGB37 having a restriction map as shown in Figure 10 and pGB42 having a restriction map as shown in Figure 10.

## Patentansprüche

1. Chinahamster-Ovarien (CHO)-Zelle, umfassend eine Nucleinsäure, die enzymatisch aktive Glucocerebrosidase codiert, wobei die Glucocerebrosidase spezifisch an ein humanes Mannoserezeptorprotein binden kann.

2. CHO-Zelle nach Anspruch 1, wobei die Nucleinsäure DNA umfaßt, die die humane Glucocerebrosidase codiert.

3. CHO-Zelle nach Anspruch 1, wobei der DNA mindestens 50% der natürlich auftretenden Region zwischen dem Promotor der Glucocerebrosidase-codierenden DNA und der ATG-Startstelle der Glucocerebrosidase-codierenden DNA fehlen.

4. Verfahren zur Herstellung enzymatisch aktiver Glucocerebrosidase, umfassend die Schritte: Einführung einer Nucleinsäure, die humane Glucocerebrosidase codiert, in eine CHO-Zelle; Veranlassung dieser Zelle zur Expression und Sekretion von Glucocerebrosidase in ein Kulturmedium; und Reinigung der Glucocerebrosidase aus diesem Kulturmedium.

5. CHO-Zelle nach Anspruch 1, wobei die Zelle mit irgendeinem Plasmid, ausgewählt aus der Gruppe pGB20 mit einer Restriktionskarte wie in Abbildung 8 beschrieben, pGB37 mit einer Restriktionskarte wie in Abbildung 10 beschrieben und pGB42 mit einer Restriktionskarte wie in Abbildung 10 beschrieben, transformiert wurde.

6. CHO-Zelle nach Anspruch 1, wobei die Zelle mit dem Plasmid pGB34 mit einer Restriktionskarte wie in Abbildung 9 beschrieben und irgendeinem Plasmid, ausgewählt aus der Gruppe pGB20 mit einer Restriktionskarte wie in Abbildung 8 beschrieben, pGB37 mit einer Restriktionskarte wie in Abbildung 10 beschrieben und pGB42 mit einer Restriktionskarte wie in Abbildung 10 beschrieben, co-transformiert wurde.

7. Verfahren nach Anspruch 4, wobei der pH-Wert des Kulturmediums zwischen etwa pH 6,5 und pH 7,2 liegt.

8. Verfahren nach Anspruch 4, wobei der pH-Wert des Kulturmediums zwischen etwa pH 6,6 und pH 6,8 liegt.

9. Verfahren nach Anspruch 4, wobei das Kulturmedium O₂ in einer Menge unter etwa 50% Sättigung zur Aufrechterhaltung der Zellen enthält.

10. Verfahren nach Anspruch 4, wobei das Kulturmedium O₂ in einer Menge von etwa 20% Sättigung und 30% Sättigung enthält.

11. Verfahren nach Anspruch 4, wobei die Nucleinsäure ein Plasmid, ausgewählt aus der Gruppe pGB20 mit einer Restriktionskarte wie in Abbildung 8 beschrieben, pGB37 mit einer Restriktionskarte wie in Abbildung 10 beschrieben und pGB42 mit einer Restriktionskarte wie in Abbildung 10 beschrieben, ist.

## Revendications

1. Cellule d'ovaire de hamster chinois (CHO pour Chinese hamster ovary) comprenant un acide nucléique codant pour une glucosyl-céramidase active du point de vue enzymatique, dans laquelle ladite glucosyl-céramidase est capable de se lier spécifiquement à une protéine réceptrice de mannose humaine.

2. Cellule CHO selon la revendication 1, dans laquelle ledit acide nucléique comprend un ADN codant pour la glucosyl-céramidase humaine.

3. Cellule CHO selon la revendication 1, dans laquelle il manque audit ADN au moins 50% d'une région naturelle située entre le promoteur dudit ADN codant pour la glucosyl-céramidase et le site de départ ATG dudit ADN codant pour la glucosyl-céramidase.

4. Procédé de production d'une glucosyl-céramidase active du point de vue enzymatique comprenant les étapes consistant à : introduire un acide nucléique codant pour la glucosyl-céramidase humaine dans une cellule CHO, induire ladite cellule à s'exprimer et à sécréter ladite glucosyl-céramidase dans un milieu de culture, et purifier ladite glucosyl-céramidase à partir dudit milieu de culture.

5. Cellule CHO selon la revendication 1, ladite cellule étant transformée par un plasmide quelconque choisi dans l'ensemble constitué de pGB20 ayant une carte de restriction présentée dans la figure 8, pGB37 ayant une carte de restriction présentée figure 10, et pGB42 ayant une carte de restriction présentée figure 10.

6. Cellule CHO selon la revendication 1, ladite cellule étant co-transformée par le plasmide pGB34 ayant une carte de restriction présentée figure 9 et un plasmide quelconque choisi dans l'ensemble constitué de pGB20 ayant une carte de restriction présentée dans la figure 8, pGB37 ayant une carte de restriction présentée figure 10, et pGB42 ayant une carte de restriction présentée figure 10.

7. Procédé de la revendication 4, dans lequel le pH dudit milieu de culture est compris entre environ 6,5 et 7,2.

8. Procédé de la revendication 4, dans lequel le pH dudit milieu de culture est compris entre environ 6,6 et 6,8.

9. Procédé de la revendication 4, dans lequel ledit milieu de culture contient de l'O₂ en une quantité environ égale à 50% de la saturation et suffisante pour entretenir les cellules.

10. Procédé de la revendication 4, dans lequel ledit milieu de culture contient de l'O₂ en une quantité comprise entre environ 20% et environ 30% de la saturation.

11. Procédé selon la revendication 4, dans lequel ledit acide nucléique est un plasmide choisi dans l'ensemble constitué de pGB20 ayant une carte de restriction présentée dans la figure 8, pGB37 ayant une carte de restriction présentée figure 10, et pGB42 ayant une carte de restriction présentée figure 10.
